# EUROPEAN PATENT SPECIFICATION

(11) **EP 3 957 717 B1**
(45) Date of publication and mention of the grant of the patent: **27.03.2024**
(21) Application number: 20208511.4
(22) Date of filing: 18.11.2020
(51) Int. Cl.: C12N 5/0775, A61K 35/50, A61K 35/51

(54) **RAPID AND EFFICIENT METHOD FOR EXPANDING HUMAN MESENCHYMAL STEM CELLS IN VITRO AND APPLICATION THEREOF**
SCHNELLES UND EFFIZIENTES VERFAHREN ZUR EXPANSION HUMANER MESENCHYMALER STAMMZELLEN IN VITRO UND DEREN VERWENDUNG
PROCÉDÉ RAPIDE ET EFFICACE D'EXPANSION DE CELLULES SOUCHES MÉSENCHYMATEUSES HUMAINES IN VITRO ET SON APPLICATION

(30) Priority: 21.08.2020 CN 202010846476
(43) Date of publication of application: 23.02.2022
(73) Proprietor: Affiliated Hospital of Zunyi Medical University, Zunyi Guizhou (CN)
(72) Inventor: XIAO, Jianhui, Zunyi Guizhou (CN); LUO, Yi, Zunyi Guizhou (CN); ZHONG, Jianjiang, Zunyi Guizhou (CN); YU, Changyin, Zunyi Guizhou (CN)
(74) Representative: Ipey

(56) References cited:
- EP-A1- 3 957 718
- FREYTES DONALD O. ET AL: "Macrophages modulate the viability and growth of human mesenchymal stem cells", JOURNAL OF CELLULAR BIOCHEMISTRY, vol. 114, no. 1, 14 November 2012 (2012-11-14), pages 220-229, XP055798900, ISSN: 0730-2312, DOI: 10.1002/jcb.24357
- CHUNG EUNKYUNG ET AL: "Crosstalk between mesenchymal stem cells and macrophages in tissue repair", TISSUE ENGINEERING AND REGENERATIVE MEDICINE - JO'JIG GONGHAG GWA JAESAENG UIHAG, KOREAN TISSUE ENGINEERING AND REGENERATIVE MEDICINE SOCIETY - KOREA, REPUBLIC OF, KI, vol. 11, no. 6, 3 November 2014 (2014-11-03), pages 431-438, XP035411342, ISSN: 1738-2696, DOI: 10.1007/S13770-014-0072-1 [retrieved on 2014-11-03]
- YIN JAMES Q ET AL: "Manufacturing of primed mesenchymal stromal cells for therapy", NATURE BIOMEDICAL ENGINEERING, NATURE PUBLISHING GROUP UK, LONDON, vol. 3, no. 2, 28 January 2019 (2019-01-28), pages 90-104, XP036695996, DOI: 10.1038/S41551-018-0325-8 [retrieved on 2019-01-28]
- MATSUMURA ETSUKO ET AL: "Pretreatment with IL-1[beta] enhances proliferation and chondrogenic potential of synovium-derived mesenchymal stem cells", CYTOTHERAPY, vol. 19, no. 2, 1 February 2017 (2017-02-01), pages 181-193, XP055798718, GB ISSN: 1465-3249, DOI: 10.1016/j.jcyt.2016.11.004
- CEDRIC MENARD ET AL: "Clinical-Grade Mesenchymal Stromal Cells Produced Under Various Good Manufacturing Practice Processes Differ in Their Immunomodulatory Properties: Standardization of Immune Quality Controls", STEM CELLS AND DEVELOPMENT, vol. 22, no. 12, 15 June 2013 (2013-06-15) , pages 1789-1801, XP055688938, US ISSN: 1547-3287, DOI: 10.1089/scd.2012.0594
- MARTA E. CASTRO-MANRREZA ET AL: "Immunoregulation by Mesenchymal Stem Cells: Biological Aspects and Clinical Applications", JOURNAL OF IMMUNOLOGY RESEARCH, vol. 2015, 1 January 2015 (2015-01-01), pages 1-20, XP055377347, US ISSN: 2314-8861, DOI: 10.1155/2015/394917
- HONGYE FAN ET AL: "Pre-treatment with IL-1&bgr; enhances the efficacy of MSC transplantation in DSS-induced colitis", CELLULAR & MOLECULAR IMMUNOLOGY, vol. 9, no. 6, 22 October 2012 (2012-10-22), pages 473-481, XP055376522, CH ISSN: 1672-7681, DOI: 10.1038/cmi.2012.40
- FADHIL MUSTAFA ET AL: "Clinical value of peripheral blood M2/M1 like monocyte ratio in the diagnosis of breast cancer and the differentiation between benign and malignant breast tumors", ACTA BIOCHIMICA POLONICA, [Online] 3 December 2019 (2019-12-03), XP055799269, PL ISSN: 0001-527X, DOI: 10.18388/abp.2019_2855 Retrieved from the Internet: URL:https://ojs.ptbioch.edu.pl/index.php/a bp/article/view/2855/4234> [retrieved on 2021-04-28]

## Description

### FIELD OF THE APPLICATION

The present invention belongs to the field of stem cells and regenerative medicine, in particular, to a rapid and efficient method for expanding human mesenchymal stem cells in vitro and an application thereof.

### BACKGROUND TECHNOLOGY

Human mesenchymal stem cells (MSC) have excellent functional characteristics such as multidirectional differentiation, immune regulation, and promotion of tissue regeneration and repair, with the advantages of low immunogenicity and no tumorigenicity, and are considered to be the most promising resource for stem cell therapy for a variety of incurable diseases such as cardiovascular diseases (Micro circulation. 2017 ; 24(1)), diabetes (Int Immunopharmacol. 2017; 44:191-196), neurological diseases (J Tissue Eng Regen Med. 2013, 7:169-82.) and malignant tumors (Cell Research. 2008; 18: 500-507.). As of 2018, more than 700 human MSC treatment clinical trials (https://www.clinicaltrials.gov) have been approved worldwide, involving hundreds of diseases. Since the first human MSC clinical trial was carried out in 1995, there have been no successful cases of large-scale clinical application. The reasons involving major basic scientific issues and key technologies related to the clinical transformation of human MSC are pending. For example, the number and quality of cells are the source, fundamental and basic issues that plague the clinical transformation of human MSC. The number of MSCs derived from human tissues is limited, which is difficult to meet the requirements of the number of cells required for clinical treatment. Therefore, the in vitro expansion of human MSCs has become a hot issue in the field of regenerative medicine. In recent years, with the rapid development of biotechnology, the expansion of human MSCs through 2D and 3D technologies such as improved culture conditions, bioreactors, microcarriers, and scaffolds has basically solved the problem of limited human MSC cells (Nature Biomedical Engineering 2019; 3:90-104). However, in the process of long-term subculture and in vitro expansion of human MSCs, problems such as abnormal cell morphology, changes in gene protein expression profile, and cell aging caused by environmental stress and physiological factors are faced with, leading to the progressive loss of biological characteristics such as human MSC cell surface markers, stem cell markers, immune regulation capabilities, migration and homing capabilities, self-renewal capabilities, and multi-directional differentiation potential, which seriously affects the quality of cells obtained by expansion, thereby profoundly affecting the clinical treatment effect and resulting in the human MSC obtained by the existing in vitro expansion technology being greatly restricted in scientific research and clinical application.

Therefore, scientists have done a lot of research work to improve the quality of human MSC expanded cells in vitro, and formulated a series of in vitro expansion strategies. For example, laser-irradiated pretreatment of culture medium, addition of exogenous cytokines and extracellular matrix (Biomaterials. 2012; 33: 4480-9; FASEB J. 2011; 25: 1474-85), treatment of natural small molecule compounds (Theranostics. 2016; 6:, 1899-1917.) and genetic engineering (Nature. 2008; 451:141-6.) have been used to maintain or enhance the stem characteristics and function of MSCs after long-term expansion. However, many innate flaws in design of these strategies limit their clinical application. For example, exogenous cytokines and extracellular matrix need to act continuously, leading to low efficiency and high cost; MSCs through genetic recombination have potential risks of mutation or malformation (J Thorac Cardiovasc Surg. 2008; 136: 1388-9.). Therefore, there is an urgent need to find a more effective and reliable method to maintain or enhance the functionality and safety of human MSCs expanded in vitro.

Inflammation is the basis of many chronic and degenerative diseases, as well as an important defense response of the body. In addition, a variety of cytokines produced during inflammation can regulate the regeneration process, remove damaged cells and initiate tissue repair, which is an important driving factor for tissue cell regeneration. For example, in the intestine, activated immune cells produce many inflammatory cytokines, including TNF-α, IL-6, IL-10 and IL-17, etc., which can control the regeneration response of intestinal cells by regulating the expansion and differentiation of intestinal stem cells; further, the production of inflammatory cytokines can prevent intestinal mucosal cells from being damaged and repairing necrotizing enterocolitis caused by aplastic disorders (N Engl J Med. 2011, 364:255-64; Cell. 2004, 23; 118: 229-41.). This shows that there are inextricable links between inflammation and tissue regeneration. In recent years, a large amount of evidence has shown that the body microenvironment is one of the important factors that determine the clinical efficacy of human MSC. It has been proven that pretreatment of inflammatory factors (TNF-α and IL-1β) can promote MSCs to secrete cytokines (IL-1A, RANTES, G-CSF) and enhance their immune regulation ability (Cell Mol Immunol. 2012; 9:473-81; Cytotherapy, 2017; 19: 181-193). The immune regulation ability of MSC is closely related to its stem characteristics; for example, Shuai et al. confirmed that melatonin can effectively prevent the lack of stem characteristics of rat bone marrow MSCs during in vitro expansion, thereby retaining the effect of in vivo immunotherapy (Theranostics. 2016, 6: 1899-1917.). This suggests that in the process of MSC expansion and culture in vitro, an appropriate inflammatory microenvironment is a potential measure to reduce the loss of stem characteristics, or even enhance the stem characteristics.

### Summary of Invention

Based on the common problems of insufficient quantity, poor quality, and limited clinical efficacy of human MSCs expanded in vitro, an object of the present invention is to provide a rapid, efficient, safe and operable method for promoting long-term expansion of human mesenchymal stem cells in vitro, so as to obtain high-quality, safe and effective human mesenchymal stem cells.

To achieve the above object, in the present disclosure human MSC and human peripheral blood immune cells such as PBMC, or PBM and PBL separated from PBMC are co-cultured, and experimental data show that immune cells may promote the expansion of human MSC, wherein the expansion effect lies in that PBL>PBMC>PBM.

The following documents all relate to culturing immune cells with mesenchymal stem cells.

D1 FREYTES DONALD O . ET AL: "Macrophages modulate the viability and growth of human mesenchymal stem cells", JOURNAL OF CELLULAR BIOCHEMISTRY, vol. 114, no. 1, 14 November 2012 (2012-11-14), pages 220-229, XP055798900 , ISSN: 0730-2312, DOI: 10.1002 /jcb.24357. D1 describes a study wherein mesenchymal stem cells (MSCs) delivered into the injured myocardium as reparative cells were subjected to the effects of polarized macrophages and the inflammatory milieu. Human MSCs were also studied using an in vitro platform with individual and combined M1 and M2 cytokines: IL-1b, IL-6, TNF-a, and IFN-g (for M1), and IL-10, TGF-b1, TGF-b3, and VEGF (for M2). Indirect and direct co-cultures were conducted using M1 and M2 polarized human THP-1 monocytes. M2 macrophages and their associated cytokines supported the growth of hMSCs. The monocytes/ macrophages derived from a monocytic cell line were used in a ratio of 1:1.

D2 CHUNG EUNKYUNG ET AL: "Crosstalk between mesenchymal stem cells and macrophages in tissue repair ", TISSUE ENGINEERING AND REGENERATIVE MEDICINE - JO'JIG GONGHAG GWA JAESAENG UIHAG, KOREAN TISSUE ENGINEERING AND REGENERATIVE MEDICINE SOCIETY - KOREA , REPUBLIC OF, Kl, vol. 11, no. 6, 3 November 2014 (2014-11-03) , pages 431-438, XP035411342 , ISSN: 1738-2696, DOI: 10.1007 /S13770-014-0072-1.

D2 is a review citing several studies on the interaction between macrophages and MSCs including D1. It also discloses observation defining macrophage- associated MSCs in coculture systems as pro-inflammatory MSCs with enhanced migratory capacity. They were used to stimulate MSC motility and to switch cytokine expression into pro-inflammatory profiles. The expression of the proinflammatory cytokines IL-6 and CXCL10 was significantly elevated in macrophage-associated MSCs.

D3 YIN JAMES Q ET AL: "Manufacturing of primed mesenchymal stromal cells for therapy", NATURE BIOMEDICAL ENGINEERING, NATURE PUBLISHING GROUP UK, LONDON, vol. 3, no. 2, 28 January 2019 (2019-01-28), pages 90-104, XP036695996 DOI: 10.1038 / S41551-018-0325-8. D3 is a review about the manufacturing of primed mesenchymal stromal cells for therapy. It describes aspects such as media, substrates, oxygenation , culture devices, and cryopreservation. Typical surface markers, differentiation and ageing are also discussed. The influence of MSCs on immune cells is also described. Although the priming of MSCs using cytokines is cited a coculture for expansion based on immune cells is not mentioned.

D4 MATSUMURA ETSUKO ET AL: "Pretreatment with II,-1[beta] enhances proliferation and chondrogenic potential of synovium-derived mesenchymal stem cells", CYTOTHERAPY, vol. 19, no. 2, 1 February 2017 (2017-02-01) , pages 181-193, XP055798718, GB, ISSN: 1465-3249, DOI: 10.1016/j.jcy t. 2016.11 .004. D4 describes that pretreatment with IL-1 enhances proliferation and chondrogenic potential of synovium-derived mesenchymal stem cells.

D5 CEDRIC MENARD ET AL: "Clinical-Grade Mesenchymal Stromal Cells Produced Under Various Good Manufacturing Practice Processes Differ in Their lmmunomodulatory Properties: Standardization of Immune Quality Controls", STEM CELLS AND DEVELOPMENT, vol. 22, no. 12, 15 June 2013 (2013-06-15), pages 1789-1801 , XP055688938, US ISSN: 1547-3287, DOI: 10.1089/scd.2012.0594. D5 discloses a comparison of immune modulatory properties of clinical-grade MSCs using a combination of fully standardized in vitro assays. BMMSCs expanded with FCS (BMMSC-FCS) or PL (BMMSC-PL), and AOSC-PL were analyzed in quantitative phenotypic and functional experiments, including their capacity to inhibit the proliferation of T, B, and NK cells. MSCs were expanded under several conditions including FBS, SA and PL and cytokines. Co-culture was used in order to assess the proliferation of the immune cells.

D6 MARTA E.CASTRO-MANRREZA ET AL: "Immunoregulation by Mesenchymal Stem Cells: Biological Aspects and Clinical Applications" , JOURNAL OF IMMUNOLOGY RESEARCH , vol. 2015, 1 January 2015 (2015-01-01), pages 1-20, XP055377347, US ISSN: 2314-8861, DOI: 10.1155/2015/394917. D6 is a review on immunoregulation by MSCs. In order to gain immunomodulatory capacity MSCs must be activated by proinflammatory cytokines such as IFN. MSCs can inhibit OCs maturation and thus prevent the activation of T lymphocytes and even more and decrease the proliferation and cytotoxic activity of NK cells. For example MSC were shown to influence the proliferation of T cells activated by PHA. Before co-culture MSCs were irradiated.

D7 HONGYE FAN ET AL: "Pre-treatment with IL-1beta enhances the efficacy of MSC transplantation in DSS-induced colitis"; CELLULAR & MOLECULAR IMMUNOLOGY, vol. 9, no. 6, 22 October 2012 (2012-10-22), pages 473-481, XP055376522, CH ISSN: 1672-7681, DOI: 10.1038/cmi.2012.40. D7 describes that pre-treatment with IL-1beta enhances the efficacy of MSC transplantation in OSS-induced colitis.

D8 FADHIL MUSTAFA ET AL: "Clinical value of peripheral blood M2/M1 like monocyte ratio in the diagnosis of breast cancer and the differentiation between benign and malignant breast tumors", ACTA BIOCHIMICA POLONICA, 3 December 2019 (2019-12-03), XP055799269, PL ISSN: 0001-527X , DOI: 10.18388/abp.2019_2855 . D8 studied the clinical value of peripheral blood M2/M1 like monocyte ratio in the diagnosis of breast cancer and the differentiation between benign and malignant breast tumours. Circulating M2-like monocytes were also detected in the peripheral blood of healthy controls. The present invention provides a rapid and efficient method for expanding human mesenchymal stem cells, as defined in claim 1.

The human mesenchymal stem cells used according to the present invention are obtained from fresh umbilical cord, amniotic membrane and cord blood of healthy term cesarean women, including umbilical cord mesenchymal stem cells, amniotic membrane mesenchymal stem cells and cord blood mesenchymal stem cells.

For the human peripheral blood immune cells used according to the present invention, PBMC is obtained by collecting peripheral blood of the normal physical examination population and using density gradient centrifugation to obtain PBMC, and further, the PBM's characteristic to easily adhere to the plastic culture plate and grow on the wall is used to separate PBM and PBL from PBMC.

In the present invention, the human peripheral blood lymphocytes and the human MSCs are co-cultured at a cell number ratio of 100:1 to 400:1, wherein the expansion effect is the best when the ratio is 300:1.

In the present invention, the human peripheral blood lymphocyte and the human MSCs are co-cultured in a contact co-culture way.

The source of the human peripheral blood lymphocyte used according to the present invention is not restricted by age, and the contact co-culture with the human MSCs may achieve good expansion effect, and there is no statistical difference between groups of different ages.

According to the present invention, the human peripheral blood lymphocyte and the human MSCs are co-cultured in a contact way for a long time, and the number of cells obtained is more than 10 times that of common culture methods.

According to the present invention, the human peripheral blood lymphocytes and the human MSCs are co-cultured in a contact way, which may significantly enhance the expression of the stem characteristic marker Oct4 of the human MSC.

According to the present invention, the human peripheral blood lymphocytes and the human MSCs are co-cultured in a contact way, which may significantly improve the proliferation ability of the human MSC, and promote the expression of the proliferating cell nuclear antigen PCNA of the human MSC.

According to the present invention, the human peripheral blood lymphocytes and the human MSCs are co-cultured in a contact way, which may enhance the migration ability of the human MSC, wherein in the scratch experiment, the number of migrating cells increases significantly.

According to the present invention, the human peripheral blood lymphocytes and the human MSCs are co-cultured in a contact way, which may improve the self-renewal ability of the human MSC, and significantly promote the cloning of the human MSC.

According to the present invention, the human peripheral blood lymphocytes and the human MSCs are co-cultured in a contact way, which may improve the multidirectional differentiation ability of the human MSC, including differentiation into osteoblasts, chondrocytes and adipocytes.

According to the present invention, the human peripheral blood lymphocytes and the human MSCs are co-cultured in a contact way, resulting in the human MSC with no immune elimination response to the disease model and being safe and effective.

The expanded human MSC of the present invention may be used to treat a mouse model of ulcerative colitis, with significant effect and no immune rejection reaction. More importantly, PBMCs derived from donors of different ages are used for co-culture of the human MSCs for in vitro expansion, and there is no statistical difference, suggesting that the autologous peripheral blood immune cells of the subject may be used to expand the MSCs. Therefore, the human MSC cells expanded in vitro using the present invention have high quality and good therapeutic effect; in particular, the expansion by using the subject's own peripheral blood immune cells overcomes the potential risk of immune rejection, is more secure, and has a huge clinical application prospect.

### Brief Description of Drawings

Fig. 1 illustrates the phenotype identification of human MSC (taking human amniotic membrane MSC as an example). (A) flow cytometry to detect surface molecular markers; (B) immunocytochemical staining to detect vimentin and keratin CK19
Fig. 2 illustrates the effects on the expansion ability of the MSC detected by EDU in case where the human PBMC and the human MSC are co-cultured in a contact way at different cell ratios.
Fig. 3 illustrates the effects on the expansion ability of the MSC detected by EDU in case where the human PBMC and the human MSC are co-cultured in different ways (contact way or non-contact way).
Fig. 4 illustrates the effects on the expansion ability of the MSC detected by EDU in case where the PBMC derived from different ages and the human MSC are co-cultured in a contact way.
Fig. 5 illustrates the effects on the expansion ability of the MSC detected by EDU in case where the different immune cells in the human peripheral blood, including PBMC and its peripheral blood mononuclear cells (PBM) and peripheral blood lymphocytes (PBL), and the human MSC are co-cultured in a contact way.
Fig. 6 illustrates the effects on the expansion ability of the MSC detected by EDU in case where the PBL and the human MSC from different tissues (umbilical cord) are co-cultured in a contact way.
Fig. 7 illustrates the effects on the expansion ability of the MSC detected by EDU in case where the PBL and the human MSC from different tissues (umbilical cord blood) are co-cultured in a contact way.
Fig. 8 illustrates the effects on the number of expanded cells of human MSC in case where the PBL and the human MSC are co-cultured in a contact way for a long time and in a traditional method for 12 days.
Fig. 9 illustrates the effects on the stem factor Oct4 in the human MSC and the expression of the proliferating cell nuclear antigen PCNA detected by western blotting in case where the PBL and the human MSC are co-cultured in a contact way.
Fig. 10 illustrates the effects on the human MSC clone formation in case where the PBL and the human MSC are co-cultured in a contact way and in a traditional method for 9 days.
Fig. 11 illustrates the effects on the differentiation to osteoblasts, chondrocytes and adipocytes for the human MSC in case where the PBL and the human MSC are co-cultured in a contact way and in a traditional method.
Fig. 12 illustrates the effects on the migration ability for the human MSC detected by wound healing assay in case where the PBL and the human MSC are co-cultured in a contact way and in a traditional method.
Fig. 13 illustrates the evaluation of the efficacy of MSC transplantation in mice with ulcerative colitis in case where the PBL and the human MSC are co-cultured in a contact way and in a traditional method. (A) mouse body weight change; (B) mouse disease activity index (DAI) score; (C) pathological observation; (D) pathological score

### Detail Descriptions of Embodiments

In order to more fully explain the implementation of the present invention, and that the objectives, technical schemes and advantages of the present invention will become more apparent, the technical solutions of the present invention will be described in more detail with reference to the drawings and examples above. MSCs from different tissues have no effect on the results (see Embodiment 7), and the present embodiment mainly uses human amniotic membrane MSC to illustrate. It should be understood that the specific embodiments described herein are only for illustrating but not for limiting the present invention. In addition, the embodiment of the present invention passed the review of the ethics committee of the Affiliated Hospital of Zunyi Medical College (ethics review number: KLLY-2017-003).

Based on the relationship between inflammation and tissue regeneration, and the influence of inflammatory cytokines on the characteristics and functions of MSC, we use human MSC to co-culture with immune cells in human peripheral blood, including peripheral blood mononuclear cells (PBMC), as well as peripheral blood monocytes (PBM) and peripheral blood lymphocytes (PBL) isolated from PBMC, finding that these immune cells, especially PBL, may significantly promote the expansion of human MSC, and the biological characteristics of the expanded cells have not changed, and even the sternness characteristics, homing and migration ability, proliferation ability, self-renewal and multidirectional differentiation ability have been significantly enhanced.

### Embodiment 1: Isolation, culture and identification of human MSC

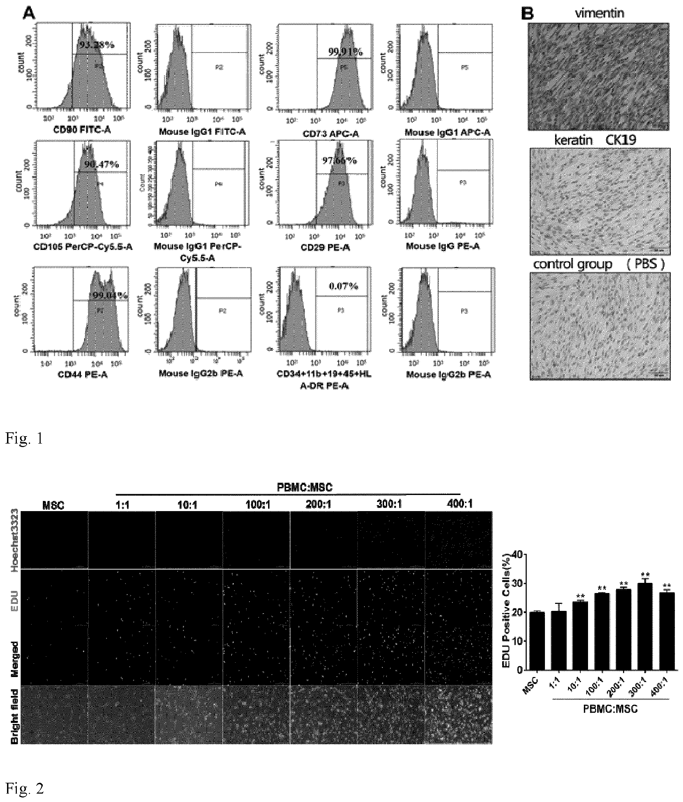

The amniotic membrane tissue is stripped from the fresh placenta of healthy term cesarean section, and the residual blood stains and mucus are repeatedly washed with D-PBS solution containing 1% bi-antibody (final concentration including penicillin of 100 IU/mL, and streptomycin of 100 IU/mL; freshly prepared before use). After cutting the amniotic membrane to pieces each with a size of about 1 cm², divided into 50 mL centrifuge tubes respectively, and then 0.05% trypsin digestion solution containing 0.02% EDTA-2Na that is about 2 times the volume of the amniotic membrane tissue is added for digesting in a constant temperature water bath at 37 °C at 185 rpm for about 30 minutes, filtering with 300 mesh stainless steel filter, and removing the supernatant; then, the above steps are repeated. The digested amniotic membrane is washed with D-PBS containing 1% double antibody once, and an equal volume of 0.5 mg/mL type II collagenase digestion solution containing 0.05 mg/mL DNase I is added for rotating and digesting at 190 rpm at 37 °C for 1 to 1.5 h until the amniotic membrane fragments are completely digested into flocculent, followed by filtering with a 300 mesh filter and collecting cell filtrate for centrifuging at 1500 rpm for 10 minutes; then, the supernatant is removed, and the cell pellet is the human amnion-derived mesenchymal stem cells (hA-MSC). The cells are resuspended in low-glucose DMEM complete medium containing 10% FBS and 10 ng/mL basic fibroblast growth factor (bFGF), plated in 75-cm² cell culture flasks, cultured at a constant temperature with 5% CO2 and 85-100% air saturated humidity at 37 °C, and subcultured when the confluence of the cells reaches 80% or more, so as to collect the passage 3 (P3) cells for experiments. In addition, human umbilical cord MSCs are isolated from fresh human umbilical cord tissue by tissue block adhesion method, and human umbilical cord blood MSCs are isolated from fresh human umbilical cord blood by density gradient centrifugation method. The obtained MSCs highly express CD90, CD105, CD73, CD44 and CD29 and other mesenchymal cell surface molecules, do not express hematopoietic stem cell markers such as CD34, CD11b, CD19, CD45 and HLA-DR and MHC class II cell surface molecules (Fig. 1A). The results of immunocytochemical staining show that the cells highly express vimentin, a mesenchymal cell marker, but do not express cytokeratin 19 (CK19), a marker of epithelial cells (Fig. 1B). They have a typical mesenchymal cell phenotype.

### Embodiment 2: Isolation of human peripheral blood immune cells

Fresh peripheral blood from a normal physical examination population is taken for diluting it with an equal volume of sterile D-PBS. An appropriate amount of Histopaque-1077 is added to a 15 mL centrifuge tube, and an equal volume of sterile D-PBS diluted blood is slowly added along the tube wall for centrifuging at 2000 rpm for 20min; the middle albuginea layer is extracted, and an equal volume of sterile D-PBS is added for centrifuging at 1500 rpm for 10 min; washing is performed repeatedly with sterile D-PBS once, and the supernatant is discarded; the cell pellet is suspended in DMEM medium containing 10% FBS and low glucose to obtain PBMC. With the characteristics of peripheral blood mononuclear cells (PBM) that are easy to adhere to and grow on plastic cell culture plates, the PBM and peripheral blood lymphocytes (PBL) are separated from the PBMC isolated above.

### Embodiment 3 Effects on the expansion ability of the human MSC detected by EDU in case where the human PBMC and the human MSC are co-cultured in a contact way at different cell ratios

The P3 of human amniotic membrane MSC in the logarithmic growth phase is taken for inoculating on a 24-well cell culture plate at a density of 3×10³/well, and the freshly-isolated PBMC is added after 16 h, wherein the density of PBMC is 3×10³, 3×10⁴, 3×10⁵, 6×10⁵, 9×10⁵ and 1.2×10⁶/well, and the PBMC and the MSC are co-cultured at cell ratios of 1:1, 10:1, 100:1, 200:1, 300:1 and 400:1. After the PBMC and the MSC are co-cultured for 48h, the expansion ability of the MSC is detected by EDU. The results are shown in Fig. 2 and Table 1. The EDU positive cell rate in the normal control MSC group (hereinafter referred to as the "MSC group") cultured by conventional methods ranges from (19.93±0.63)%; when PBMC:MSC=1:1 or 10:1, the expansion ability of MSC is not significantly enhanced; when PBMC:MSC=100:1, the EDU positive cell rate in MSC increases from (19.93±0.63)% to (26.55±0.37) )%; when PBMC:MSC=300:1, the EDU positive cell rate reaches (30.01±1.67%), which is the maximum value; however, when the cell ratio continues to increase, the expansion ability of MSC begins to decrease, e.g., the EDU positive cell rate is (26.89±0.97)% when PBMC:MSC=400:1. Therefore, when the PBMC and the MSC are co-cultured at a cell ratio of 100:1 to 400:1, the expansion ability of the MSC may be significantly improved, wherein when the two are co-cultured at a cell ratio of 300:1, the human MSC has the strongest expansion ability and the best promoting effect on expansion.

**Table 1 Effects on the expansion ability of the MSC detected by EDU in case where the contact co-culture is performed at different cell ratios**

| Cell ratios (PBMC:MSC) | EDU positive cell rate (%) |
|---|---|
| 1:1 | 20.25±2.78 |
| 10:1 | 23.43±0.75** |
| 100:1 | 26.55±0.37** |
| 200:1 | 27.93±0.69** |
| 300:1 | 30.01±1.67** |
| 400:1 | 26.89±0.97** |

| | |
|---|---|
| Note: The EDU positive cell rate in MSC group is (19.93±0.63)%. Compared with MSC, ***p*<0.01. | |

### Embodiment 4 Effects on the expansion ability of the MSC in case where the human PBMC and the human MSC are co-cultured in different ways (contact way or non-contact way)

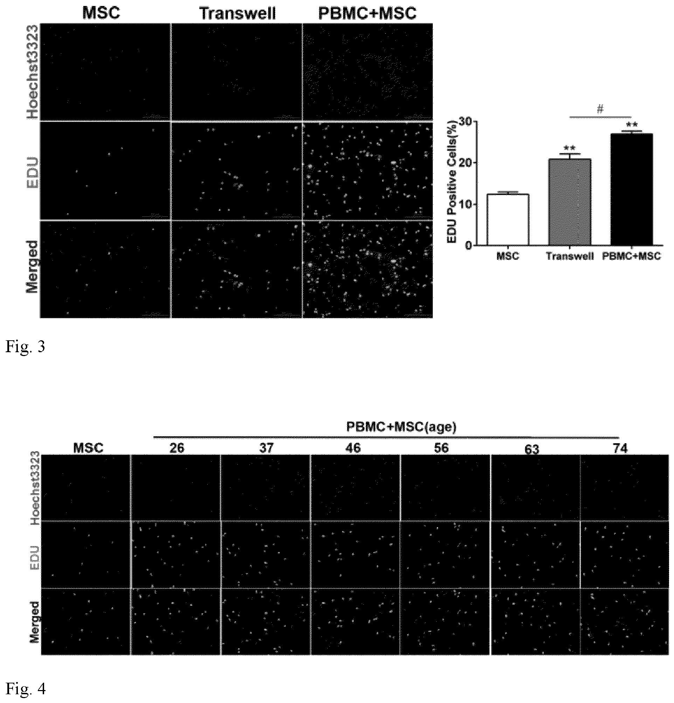

The P3 of human amniotic membrane MSC in the logarithmic growth phase is inoculated into the upper chamber of Transwell (Corning, 3470) at a density of 10³/well, and 10⁵ freshly-separated PBMCs in the lower or upper chambers to be co-cultured with MSCs; after 48 hours, the upper chamber is taken out to test the expansion ability of MSC detected by EDU. The results are shown in Fig. 3, the EDU positive cell rate in the normal control MSC group is (12.43±1.02)%; both the contact and non-contact co-culture of PBMC and MSC may significantly improve the expansion ability of MSC, but the contact way has the better effects, resulting in the EDU positive cell rate of (26.97±1.22)% (Table 2).

**Table 2 Effects on the expansion ability of the MSC detected by EDU in case where co-cultured in different ways (contact way and non-contact way).**

| co-culture way of cells | EDU positive cell rate (%) |
|---|---|
| non-contact co-culture (Transwell) | 20.84±2.23* |
| contact co-culture (PBMC+MSC) | 26.97±1.22** |

| | |
|---|---|
| Note: The EDU positive cell number in MSC group is (12.43±1.02)%. Compared with MSC, ***p*<0.01. | |

### Embodiment 5 Effects of PBMC from different donor ages on the expansion of human MSC

The P3 human amniotic membrane MSCs in the logarithmic growth phase are seeded on a 24-well cell culture plate at a density of 3×10³/well, and the freshly-separated PBMCs from different age groups are added after 16 h. After the PBMC and the MSC are co-cultured for 48 h, the expansion ability of the MSC is detected by EDU. The results (Fig. 4) show that the EDU positive cell rate in the normal control MSC group is (19.82±3.58)%, and compared with the MSC group, the PBMCs derived from different donor ages may significantly enhance the expansion ability of MSCs with an average range of the EDU positive cell rate between (25.54±3.08)% and (26.89±5.48)% (Table 3), but the PBMCs from different donor ages have no statistical difference in the expansion effect of MSC.

**Table 3 Effects on the expansion ability of the MSC detected by EDU in case where the PBMC derived from different donor ages and the human MSC are co-cultured in a contact way.**

| age group (years) | number of cases | EDU positive cell rate (%) |
|---|---|---|
| 21-30 | 10 | 25.54±3.08** |
| 31-40 | 10 | 26.80±4.96** |
| 41-50 | 10 | 26.89±5.48** |
| 51-60 | 10 | 26.76±5.53** |
| 61-70 | 10 | 26.80±3.44** |
| 71-80 | 10 | 26.54±4.20** |

| | | |
|---|---|---|
| Note: The EDU positive cell number in MSC group is (19.82±3.58)%. Compared with MSC, ***p*<0.01. | | |

### Embodiment 6 Effects on the expansion ability of the MSC in case where different immune cells derived from human peripheral blood and the human MSC are co-cultured in a contact way

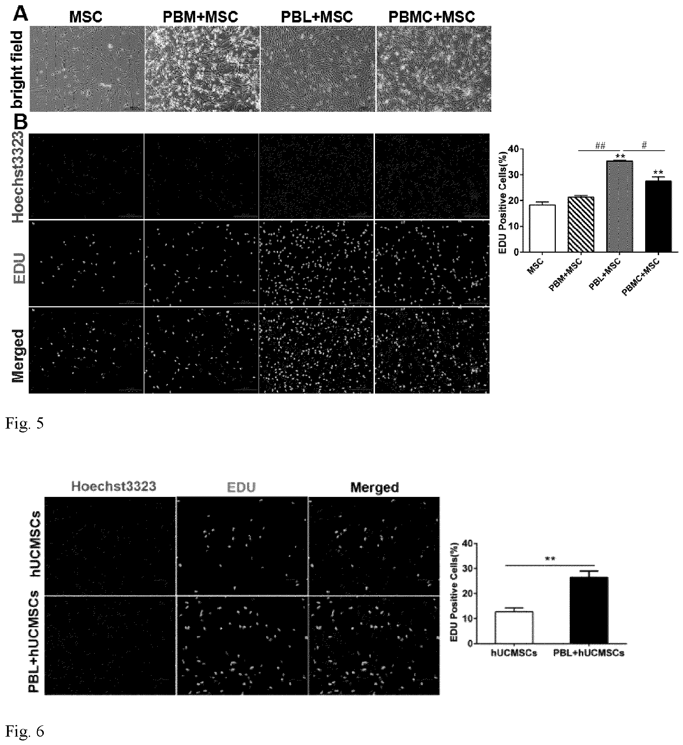

Density gradient centrifugation method is used to separate PBMC from normal human peripheral blood, and seeded on a 24-well cell culture plate at a density of 3×10⁵/well; after 2h, with the characteristics of PBM that it is easy to adhere to the plastic culture plate to grow on the wall, the PBM and the PBL are separated from the PBMC. In addition, the third generation of human amniotic membrane MSCs in the logarithmic growth phase are inoculated at a density of 3×10³/well into 24-well cell culture plates which containing PBMC, PBM and PBL respectively for co-culturing for 48h, then the morphological characteristics of MSCs are observed under a microscope, and the effects of PBMC, PBM and PBL on the expansion of MSCs are detected by EDU. The results show that after PBMC, PBM, PBL and MSC are co-cultured, MSCs all grew in a long spindle-shaped spiral, but in terms of the cell density, PBL>PBMC>PBM (Fig. 5A). The EDU experiment shows that the normal control MSC group is (18.31±2.01)%, and the co-culture of PBMC, PBM, PBL and MSC may increase the proportion of EDU positive cells to varying degrees with a range from (21.31±1.04) % to ( 35.21±0.51) % (Table 4); in terms of the ability of promoting expansion, PBL>PBMC>PBM, wherein the PBL has the best effects (Fig. 5B).

**Table 4 Effects on the expansion ability of the MSC in case where different immune cells derived from human peripheral blood and the human MSC are co-cultured in a contact way**

| groups | EDU positive cell rate (%) |
|---|---|
| normal control MSC group | 18.31±2.01 |
| monocyte co-culture group (PBM+MSC) | 21.31±1.04 |
| lymphocyte co-culture group (PBL+MSC) | 35.21±0.51^{∗∗#} |
| mononuclear cell co-culture group (PBMC+MSC) | 27.50±1.10^{∗∗} |

| | |
|---|---|
| Note: Compared with the MSC group, **p<0.01; compared with the PBMC+MSC, ^{#}*p*<0.05. | |

### Embodiment 7 Effects on the expansion ability of the MSC detected by EDU in case where the PBL and the MSC from different tissue sources (umbilical cord and umbilical cord blood) are co-cultured in a contact way.

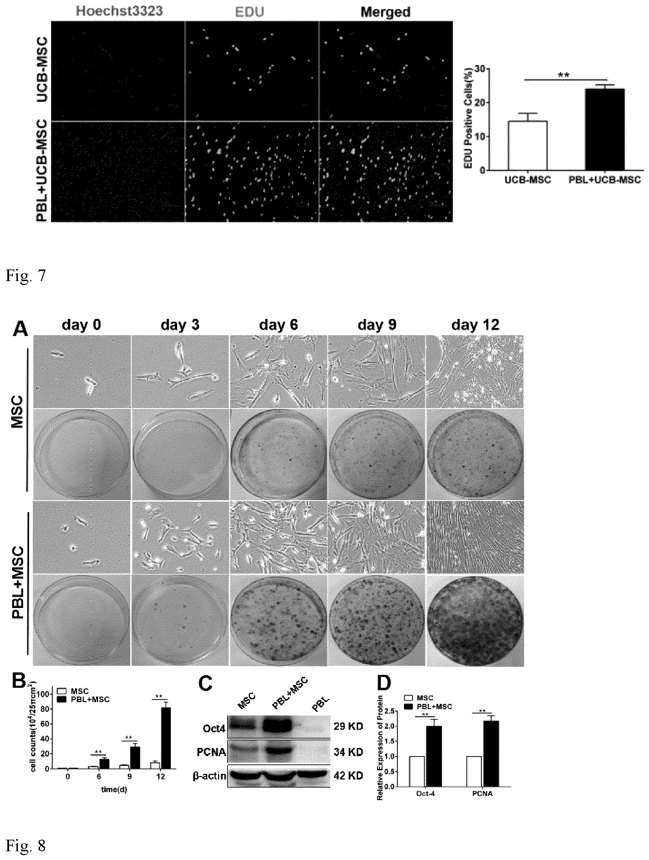

The human umbilical cord-derived mesenchymal stem cells (hUC-MSC) are separated from fresh umbilical cord tissue using the tissue block adhesion method, and the human umbilical cord blood-derived mesenchymal stem cells (hUCB-MSC) are separated from fresh cord blood by the density gradient centrifugation; all are purified by trypsin, and the third generation cells are collected for experiments. The P3 hUC-MSC and hUCB-MSC in the logarithmic growth phase is taken for inoculating in a 24-well cell culture plate at a density of 3×10³/well, and the freshly-separated PBL is added after 16h for co-culturing for 48h; then, EDU is used to detect the expansion ability of hUC-MSC and hUCB-MSC. The results show that similar to hA-MSC derived from PBL and human amniotic membrane tissue (Figure 5), the contact co-culture of PBL and hUCMSC may significantly promote the expansion of hUC-MSC (Fig. 6); similarly, the contact co-culture of PBL and hUCB-MSC may also significantly promote the expansion of hUCB-MSC (Fig. 7). Therefore, the human peripheral blood immune cells PBL may enhance the expansion ability of MSCs through the contact co-culture, which is not limited by the tissue source of MSCs, and has a significant effect in promoting expansion on umbilical cord tissue and blood-derived MSCs. The following mainly takes hA-MSC as an example to carry out PBL co-culture to evaluate its biological characteristics and functions.

### Embodiment 8 Effects on the expansion effect of MSC in case where the PBL and the MSC are co-cultured in the contact way in vitro for a long time

The P3 of human amniotic membrane MSCs in logarithmic growth phase are taken for inoculating in cell culture dishes with diameter of 10 cm at a density of 10⁴ per dish respectively, and the freshly-separately PBL is added after 16h for crystal violet staining and photographing on the 3rd, 6th, 9th and 12th day of the co-culture respectively; on the 6th, 9th and 12th day of the co-culture, the cells are digested with trypsin and centrifuged to collect the cells, and the cells are counted with a cell counter respectively; on the 12th day of co-culture, the total cell protein is extracted, and Western blotting is used to detect the expression levels of the proliferating cell nuclear antigen PCNA and the stem characteristic transcription factor Oct4. The results show that compared with the expansion of MSC alone, when PBL is co-cultured and expanded with MSC for 3 days, the number of MSC cells begin to increase significantly, about 4 times on the 6th day, more than 7 times on the 9th day, and more than 10 times on the 12th day (Table 5, Fig. 8A, B); in addition, when PBL and MSC are co-cultured for 12 d, the expression levels of PCNA and stem characteristic transcription factor Oct4 in MSC are significantly increased (Figs. 8C, D).

**Table 5 Effects on the expanded cell number of MSC in case where the PBL and the MSC are co-cultured in the contact way in vitro for a long time**

| groups | time (day) | cell number (×10⁴) |
|---|---|---|
| MSC | 0 | 1 |
| | 6 | 3.17±0.63 |
| | 9 | 4.92±0.38 |
| | 12 | 8.17±2.47 |
| PBL+MSC | 0 | 1 |
| | 6 | 12.83±2.08^{∗∗} |
| | 9 | 29.17±4.73^{∗∗} |
| | 12 | 81.67±7.64^{∗∗} |

| | | |
|---|---|---|
| Note: Compared with MSC, **p<0.01. | | |

### Embodiment 9 Effects on the proliferating cell nuclear antigen PCNA and the stem characteristic transcription factor Oct4 of MSC in case where the PBL and the MSC are co-cultured in a contact way

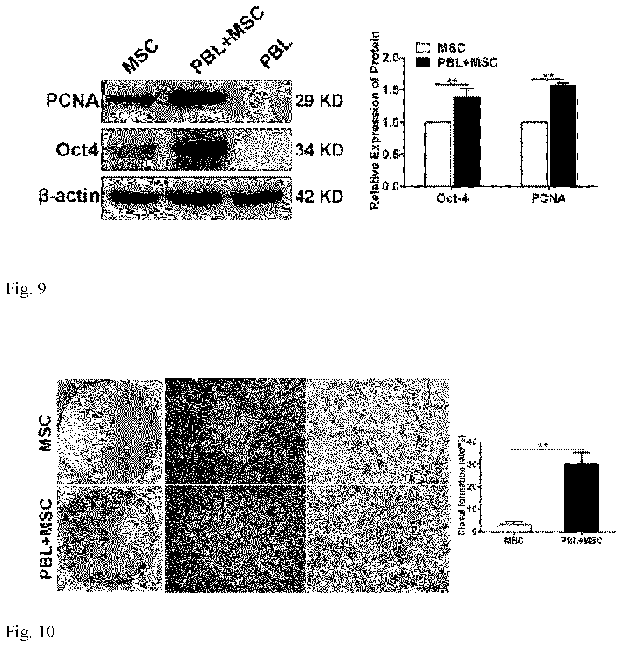

The P3 of human amniotic membrane MSCs in logarithmic growth phase are taken for inoculating in a cell culture dish with a diameter of 10 cm at a density of 2×10⁵, and the freshly-separately PBL is added after 16 h for co-culturing for 48 h; then the total cell protein is extracted, and Western blotting is used to analyze the expression of the proliferating cell nuclear antigen PCNA and the stem characteristic transcription factor Oct4. The results (Fig. 9) show that after the MSC and the PBL are co-cultured, the expression level of the proliferating cell nuclear antigen PCNA and the stem characteristic transcription factor Oct4 of MSC may be significantly enhanced.

### Embodiment 10 Effects on the self-renewal ability of MSC in case where the PBL and the MSC are co-cultured in the contact way

The P3 human amniotic membrane MSC in the logarithmic growth phase is taken for inoculating in a 6-well cell culture plate at a density of 100 cells/well, and the freshly-separated PBL is added after 16h for co-culturing for 9 days; then, the crystal violet staining is used to observe the formation of cell colonies, and photographs are taken and recorded. The results (Fig. 10) show that after 9 days of co-culture of MSC and PBL, the colony formation rate of MSC is 33.3%, which is more than 10 times higher than that of the conventional culture control group (3.3%).

### Embodiment 11 Effects on the differentiation ability of MSC in case where the PBL and the MSC are co-cultured in the contact way

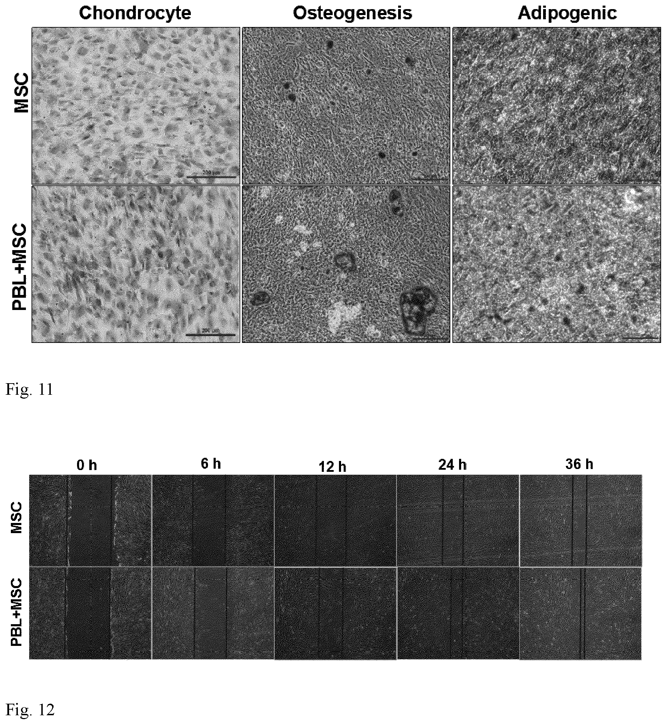

The P3 human amniotic membrane MSC in the logarithmic growth phase is taken for inoculating in a 6-well cell culture plate at a density of 2×10⁴ cells/well, and the freshly-separated PBL is added after 16h for co-culturing for 48h; when the cell confluence reaches 80%, replace with osteogenic, adipogenic, and chondrogenic differentiation medium. During the whole process of induction and differentiation, the medium is changed every 3 days, and on 21th day, toluidine blue staining is used to detect the production of glycosaminoglycans in the extracellular matrix of chondrogenic differentiation, alizarin red S staining is used to determine the formation of osteogenic differentiation calcified nodules, and oil red O staining is used to determine the formation of adipogenic lipid droplets. The results (Fig. 11) show that compared with MSCs obtained by conventional culture, the MSCs obtained by co-culturing PBL and MSC in a contact way have stronger cartilage matrix glycosaminoglycan production ability, calcium nodule formation ability and lipid droplet production ability. It is suggested that the contact co-culture of PBL and MSC has the ability to enhance MSC differentiation into osteogenic, adipogenic and chondrogenic differentiation.

### Embodiment 12 Effects on the migration ability of MSC in case where the PBL and the MSC are co-cultured in the contact way

The P3 human amniotic membrane MSC in the logarithmic growth phase is taken for inoculating in a 6-well cell culture plate at a density of 5×10⁴ cells/well, and the freshly-separated PBL is added after 16h for co-culturing until the cell confluence reaches 100%, followed by scoring the culture plate with 200 µL sterile pipette tip, washing away cell debris with sterile PBS, and placing in a 37°C, 5% CO2 incubator with saturated humidity; then, the same scratch position is photographed and recorded at 0, 6, 12, 24, and 36 h of co-culture. The results are shown in Fig. 12, and the contact co-culture of the PBL and the MSC may significantly enhance the migration ability of MSC.

### Embodiment 13 Effects on the therapeutic effect of MSC in case where the PBL and the MSC are co-cultured in the contact way

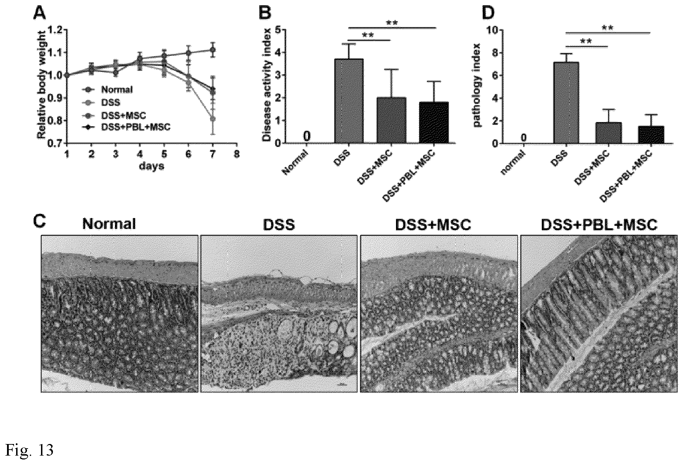

A mouse model of ulcerative colitis is constructed by free diet 3% DSS, and C57 mice aged 5-7 weeks are randomly divided into 4 groups, a total of 40 mice, 10 mice in each group, which are marked with Normal, DSS, DSS+MSC and DSS+PBL+MSC respectively. The DSS+MSC group and the DSS+PBL+MSC group are treated with P4 and lymphocytes by intraperitoneal injection of P4 MSCs (1×10⁷/head) after 48 hours at the start of feeding DSS. Normal and DSS mice are injected with equal volume of sterile PBS at the same time. During the observation, the weight is regularly measured and the death situation is recorded every day, as well as observing whether there are loose stools, bloody stools, etc. The disease activity index (DAI) is calculated based on the weight, diarrhea, blood in the stool, and death of the mouse. Ten days after injection of human amniotic membrane MSC, the mice are sacrificed, and the colon segment is fixed with 4% paraformaldehyde overnight at room temperature, embedded in paraffin, and cut into 4 µm sections for HE staining. The inflammatory cell infiltration and crypt and goblet cell structure of colon tissue are observed under a microscope, and histopathological score is performed according to the previously reported method. The results show (Fig. 13) that on the 5th day of feeding DSS, the mouse begin to show severe inflammatory colitis symptoms, including weight loss, loose stools, bloody stools, etc., and die on the 7th day. Histological analysis shows (Figs. 13C and D) that DSS feeding causes severe inflammation and damage to the colonic mucosa of mice. After intravenous injection of P4 MSC obtained by co-culture of PBL and P3 MSC, similar to intravenous injection of P4 MSC without immune cell treatment, no immune exclusion reaction occurs, which may significantly reduce DSS-induced weight loss, bloody stool, colon inflammation and damage, and effectively prevent the death of colitis rats (Fig. 13), suggesting that the MSCs obtained by co-culture and expansion of PBL and MSCs are safe and effective for the treatment of the disease model.

## Claims

1. A rapid and efficient method for expanding human mesenchymal stem cells *in vitro,* comprising co-culturing immune cells derived from human peripheral blood comprising human peripheral blood lymphocytes, with human mesenchymal stem cells, the co-culturing being in contact mode at a cell number ratio of the human peripheral blood lymphocytes to the human mesenchymal stem cells of 100 to 400:1.

2. The method according to claim 1, wherein the cell number ratio is 300:1.

## Patentansprüche

1. Schnelles und effizientes Verfahren zur Expansion humaner mesenchymaler Stammzellen *in vitro,* umfassend das Co-Kultivieren von Immunzellen, die aus humanem peripherem Blut abgeleitet sind, das Lymphozyten aus humanem peripherem Blut umfasst, mit humanen mesenchymalen Stammzellen, wobei das Co-Kultivieren im Kontaktmodus bei einem Zellzahlverhältnis der Lymphozyten aus humanem peripherem Blut zu den humanen mesenchymalen Stammzellen von 100 bis 400:1 erfolgt.

2. Verfahren nach Anspruch 1, wobei das Zellzahlverhältnis 300:1 beträgt.

## Revendications

1. Procédé rapide et efficace d'expansion de cellules souches mésenchymateuses humaines *in vitro,* comprenant la coculture de cellules immunitaires dérivées de sang périphérique humain comprenant des lymphocytes de sang périphérique humain, avec des cellules souches mésenchymateuses humaines, la coculture étant dans un mode contact à un rapport de nombre de cellules entre lymphocytes de sang périphérique humain et cellules souches mésenchymateuses humaines de 100 à 400:1.

2. Procédé selon la revendication 1, dans lequel le rapport du nombre de cellules est de 300:1.
